# EUROPEAN PATENT SPECIFICATION

(11) **EP 2 254 477 B1**
(45) Date of publication and mention of the grant of the patent: **29.05.2013**
(21) Application number: 08833737.3
(22) Date of filing: 29.09.2008
(51) Int. Cl.: A61N 7/02, A61B 17/00, A61N 7/00

(54) **HANDHELD TRANSDUCER SCANNING SPEED GUIDES AND POSITION DETECTORS**
HAND-WANDLER-SCANNINGGESCHWINDIGKEITSFÜHRUNGEN UND POSITIONSDETEKTOREN
GUIDES DE LA VITESSE DE BALAYAGE ET DETECTEURS DE POSITION D'UN TRANSDUCTEUR PORTATIF

(30) Priority: 28.09.2007 US 995895 P
(43) Date of publication of application: 01.12.2010
(73) Proprietor: Nivasonix, LLC., Carpinteria, CA 93013 (US)
(72) Inventor: PEDERSEN, Laust, G., Santa Barbara CA 93105 (US); DAVLANTES, Constantine, C., Carpinteria CA 93013 (US)
(74) Representative: Zenz
(86) International application number: PCT/US2008/078188
(87) International publication number: WO 2009/043046

(56) References cited:
- WO-A2-2005/006954
- US-A- 3 964 297
- US-A- 5 645 066
- US-A- 6 132 379
- US-A1- 2003 130 615
- US-B1- 6 213 781
- US-B2- 7 258 674

## Description

### BACKGROUND OF THE INVENTION

### 1. Field of the Invention

The present invention relates to the field of non-invasive external ultrasound lipoplasty, skin tightening, and various non-invasive aesthetic, dermatologic, and therapeutic applications.

### 2. Prior Art

During a non-invasive external ultrasound lipoplasty, skin tightening, aesthetic, dermatologic, or other therapeutic procedure with a handheld transducer, it becomes particularly important to apply and distribute the ultrasound energy dose according to the amount and location of the fat to be emulsified, the degree and location of skin tightening needed, or the extent and type of aesthetic, dermatologic, or other therapeutic desired effect.

For this purpose the transducer's movement or instantaneous scanning speed needs to be known or better yet its position from which the scanning speed can be easily derived.

U.S. Patent No. 7,347,855 teaches a passive system of computerized tracking of a multiplicity of target volumes with compensation for body movements.

U.S. Patent No. 6,645,162 teaches an active tracking system depicted in their Figures 12 and 13 guiding a transducer in a linear motion.

"Selective Creation of Thermal Injury Zones in the Superficial Musculoaponeurotic System Using Intense Ultrasound Therapy", (Matthew W. White et al., ARCH FACIAL PLAST SURG, Vol. 9, Jan/Feb 2007) shows an ultrasound probe by Ulthera with an internal transducer performing a controlled linear motion, thereby acting as an active tracking system.

U.S. Patent No. 7,150,716 is specific to diagnostic ultrasound, and teaches two methods (and systems) of detecting transducer scanning speed, namely one through the use of an sensor similar to an optical computer mouse and another through real time de-correlation of ultrasound images.

U.S. Patent No. 5,645,066 discloses an apparatus according to the preamble of claim 1.

In the case of a handheld transducer without hardware to do the spatial and time feedback, the control has to come through the operator. It should be realized that the optimum speed of the transducer across the skin is strongly related to the optimum local exposure time. Too short an exposure time (fast motion) will not give adequate cavitation or heat (when needed) and could therefore reduce the efficacy of the procedure to near zero. Too slow a motion could create too much cavitation or heat with the potential for indiscriminant tissue destruction. In order to separate cavitation and heating further, there may be cases where multiple passes over the same area are necessary.

### BRIEF DESCRIPTION OF THE DRAWINGS

Figure 1a is a functional block diagram of one embodiment not forming part of the present invention.
Figure 1b is a functional block diagram of an alternate form of the embodiment of figure 1a.
Figure 2a is a functional block diagram of an embodiment of the present invention.
Figure 2b is a functional block diagram of alternate form of the second embodiment.
Figure 3 is a functional block diagram of still another embodiment not forming part of the present invention.
Figure 4 is a view of a graphical user interface (e.g., touch screen display menu) with a scanning speed indicator in accordance with the embodiment of Figure 1a.
Figure 5 is a transducer with an integral scanning speed indicator, which in this example consists of five LEDs, in accordance with the embodiment of Figure 1b.
Figure 6 is a flexible scanning speed guidance strip with built in LEDs in accordance with the embodiments of Figures 2a and 2b.
Figure 7 is a cross sectional view of an exemplary optical sensor in accordance with still another embodiment not forming part of the present invention.

### DETAILED DESCRIPTION OF THE PREFERRED EMBODIMENTS

The optimum transducer "scanning" speed for delivering a predetermined dose of ultrasound to a desired treatment area determined by a scanning plan is a function of both the cavitation related Mechanical Index (MI) and tissue temperature Thermal Index (TI) settings. While cavitation is a threshold mechanism there is both an amplitude factor beyond the threshold level and an exposure time factor involved in emulsifying a certain fraction of the treated fat, whereby low settings require a slow scanning speed and high settings require faster scanning speeds. The relationships can be estimated from the numerical values of MI and TI and further refined empirically using data from animal and clinical studies. Furthermore, since the transducer is not 100% energy efficient, its face (skin contact area) will create heat and if not properly controlled may present a hazard for potential skin burns. Moving the transducer across the skin surface will also significantly reduce localized peak skin temperature. In the case where there is a sensor monitoring the transducer face temperature, control of the skin heating can be included in the speed indicator. If there is no transducer face temperature sensor the suggested transducer movement speed component due to tissue heating can be based on empirical data from animal and clinical studies.

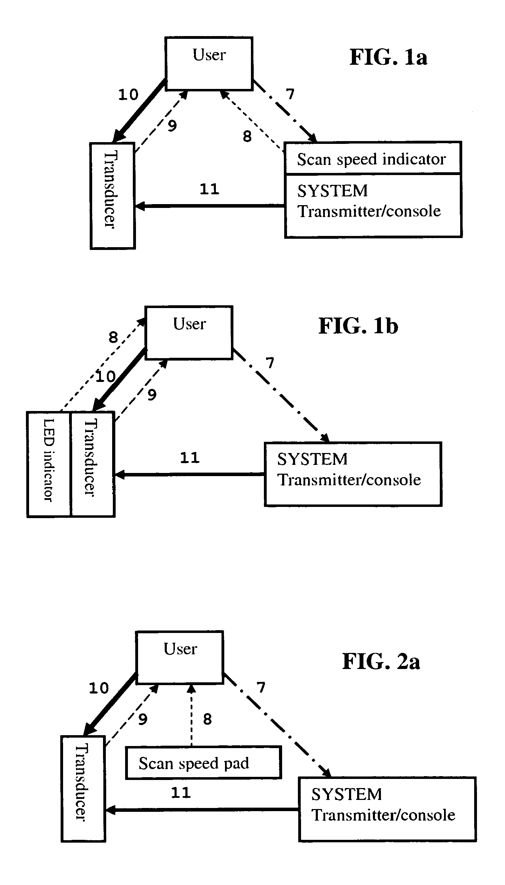

There are at least three approaches for addressing control of the ultrasound dose delivery, as summarized in Figs. 1a and 1b, Figs. 2a and 2b, and Fig. 3

One method requires the user to be part of the feedback loop, whereby the system, the transducer, or a separate device acts as a visual guide for the user to apply the desired scanning speed. The first embodiment is an example of this approach.

Another method consists of a subsystem that detects the transducer scanning velocity and in real time transfers this information to the system, which in turn adjusts parameters such as MI and TI (i.e., ultrasound dose) to achieve the desired effect based on the actual speed of transducer movement. This relieves the user from precisely matching the desired scanning speed, but still requires the user to keep track of the transducer position and the ultrasound beam focal depth. The second embodiment is an example of this.

A third method monitors the transducer position, which is transferred to the system in real time. With this information and the presence of a clock, the transducer velocity may also be easily calculated. Now the system can automatically adjust the needed parameters such as MI, TI and focal length to accomplish the planned treatment, giving the user the freedom to move the transducer almost "at will". The third embodiment is an example of this.

It should be noted that there is much more value in using a 3D coordinate system, where the (contoured) skin defines two of the dimensions and the depth below the skin surface is the third, rather than a Cartesian coordinate system fixed to the operating room or even fixed to localized patient movements.

The connecting lines in the functional block diagrams in Figures 1a to 3 have the following meaning. The occasional user control of the system/console is shown as 7. Item 8 indicates that the user reads the scanning speed indicator. Item 9 indicates that the user views the actual scanning speed of the transducer. Item 10 indicates that the user actually holds and moves the transducer. Item 11 shows the transmit signals from the system to the transducer. Item 12 shows the low level power supply and control signals from the system to the Scanning Speed Pad 5 or optical sensor. Item 13 indicates the path of sensor signals from the optical sensor to the built in Decoder, which translates the information into position and speed.

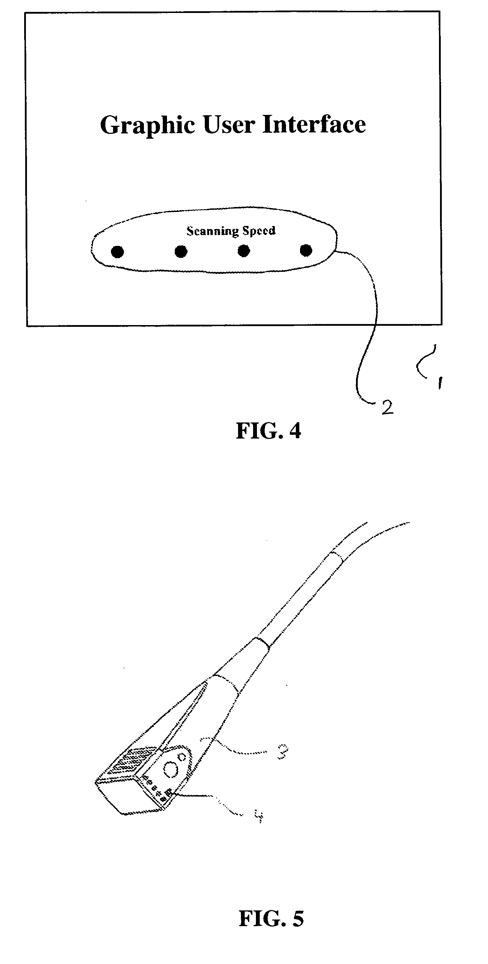

One embodiment (Figures 1a and 4) is to have a visual transducer scanning speed indicator on the transmitter (main unit, console or system) that moves with the same speed that is optimal for the transducer motion on the skin. The speed indicator can take the form of a moving cursor 2 on a screen 1 of the transmitter. The moving cursor can take many forms, the one shown in Figures 1a, 1b, 2a, 2b and 4 through 6 consists of four moving dots (light sources such as LEDs). These may be sequentially switched on and off at a controlled rate, or the first switched on, the second switched on, etc. with all being switched off and the cycle repeated after the last light source has been switched on, either of which is to be considered sequential switching on, or scanning. The user can then practice matching that speed while holding the transducer near the screen. When sufficiently proficient he/she can match the speed when scanning on the skin. As verification, the user can mark the skin for a certain distance and calculate the time needed to traverse that distance based on the numerical value of the desired velocity.

Another version (Figures 1b and 5) of the first embodiment is to have a visual transducer scanning speed indicator on the transducer itself 3 , for example in the form of an array of visual indicators (light sources) such as Light Emitting Diodes (LEDs) 4 , which light up in a sequence corresponding to the desired speed of the transducer. It will then be up to the user to provide the "feedback loop" by moving the transducer at the indicated speed. Here the user can perform the same verification as described above.

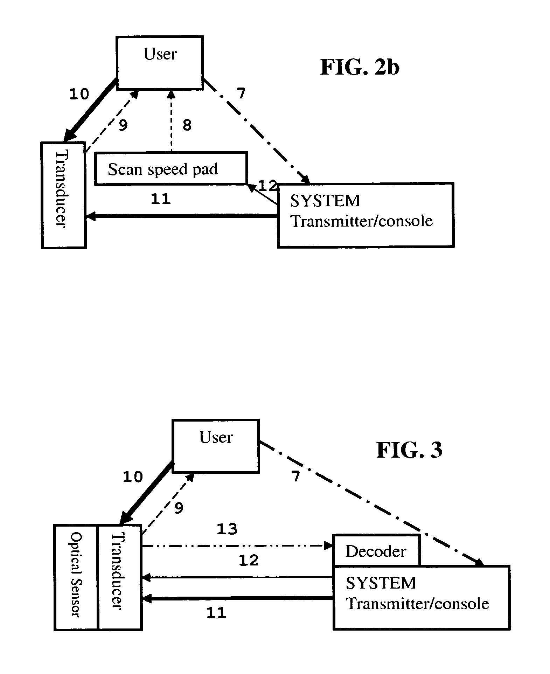

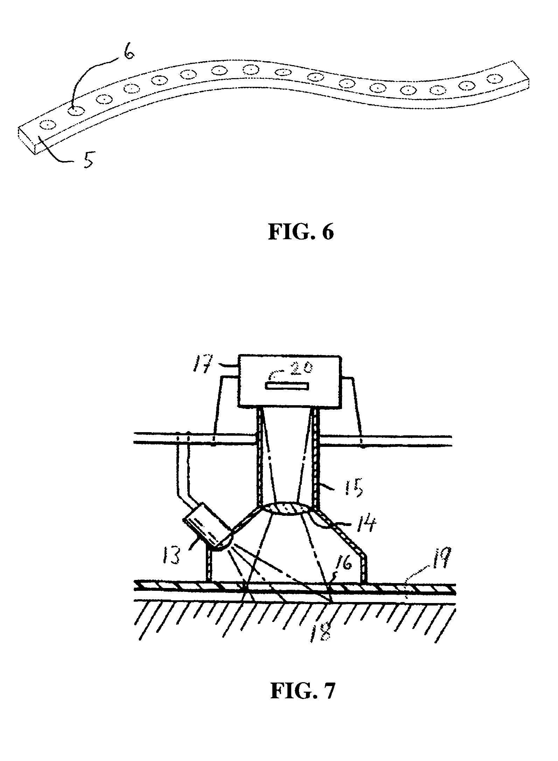

A second embodiment is a separate flexible scanning speed guidance pad 5 (Figures 2a, 2b and 6), which can be placed on the patient adjacent to the intended transducer path. The flexible material can be silicone rubber or other material with LEDs (or other visual indicators) 6 molded in. The LEDs are sequentially switched on and off so that they provide visual scanning speed guidance in proximity to the transducer. The speed guidance pad can be manufactured in different lengths and/or from different materials to fit the desired treatment area, and can also be either disposable (single patient use), semi-disposable, or reusable. The LEDs can be powered either by batteries, as in the embodiment of Figure 2a, or by the transmitter, embodiment of Figure 2b, in which case a power cord is detachably connected to the guidance pad. By being connected to the transmitter, the system may display the scanning speed, scanning location or position, and range of scan distance if the pad is physically longer than the desired scan needed to match the system settings, while the battery operated solution either would require wireless transmission of the information, or require the user to set the scanning speed according to the transmitter's displayed parameters.

A third embodiment is an optical 2D location sensor technology similar or identical to those used in an optical computer mouse, as in Figures 3 and 7. The sensor primarily consists of a light source 13, a translucent membrane or cavity 16, a lens 14 to collimate the reflected light from the skin 18, which also goes through the acoustic coupling gel 19 and continues through an optical guide to an optical sensor array 20 embedded in an integrated circuit 17. As indicated in Figure 3 the optical sensor is attached to or built into a transducer, generally like that of Figure 5. The sensor information is passed through the transducer cable and processed in the system to find the position and velocity of the transducer. Any speckle, phase shift, frequency shift or other characteristics may be used to detect motion and velocity.

Alternatively the sensor information may be wirelessly communicated to the system.

As with a computer mouse, the optical 2D location sensor can lose track of the transducer position if lifted from the surface (skin). This can be overcome with a simple calibration process, whereby the user moves the transducer to a marked calibration spot on the skin, push a calibration button on the transducer or on the system, and moves the transducer on the skin to the desired location.

In the case of a "brush-beam" (non circular symmetric beam) it becomes important to scan approximately perpendicular to the width (long axis) direction of the brush-beam.

This third embodiment is very adaptable to a scanning plan in which the user graphically composes a 3D volume using software within the system or off line, showing the relative location and amount of treatment wanted, both with respect to cavitation (fat emulsification) , heating (skin tightening) , or other aesthetic/ dermatologic/ therapeutic treatments.

Off line use of the scanning plan software allows data transfer to the system. During the procedure, the system can keep track of the transducer's location and in real time can adjust critical parameters such as MI, T I and focal depth (if equipped with electronic focusing) , so the desired treatment "dose" eventually will be delivered. The real time difference between the desired and actual delivered "dose" can also be displayed on the system graphically in a 2D format, so the user can concentrate the transducer motion in the area where more treatment is needed. This allows the user to move the transducer freely within certain boundaries with respect to both position and speed.

For the best outcome with respect to the treatment plan, the transducer needs to be oriented perpendicular to the skin and in the case of a brush-beam transducer, the scanning velocity vector needs to be perpendicular to the brush width direction. However, an angular error relative to the exact perpendicularity is a cosine function, meaning that it is a weak dependency, so that in reality, perpendicularity need not be monitored, but can be continuously estimated by the user.

The suggested speed shown by the various embodiments of the speed indicator can be based on MI, T I and instantaneous transducer face temperatures and/or acquired data from animal and clinical studies. While the above methods are intended to be used in conjunction with a non-invasive ultrasound lipoplasty transducer, the inventions, the scanning light source of the first two embodiments can be used on handheld transducers for other modalities, including aesthetic, dermatologic, or other therapeutic applications. In the claims to follow, a reference to a handheld external ultrasound treatment transducer is a reference to a handheld external ultrasound transducer useable for lipoplasty, skin tightening, aesthetic, dermatologic/, and other therapeutic purposes.

Thus, while certain preferred embodiments of the present invention have been disclosed and described herein for purposes of illustration and not for purposes of limitation, it will be understood by those skilled in the art that various changes in form and detail may be made therein without departing from the scope of the invention.

## Claims

1. Apparatus for guiding the scanning speed a user moves a handheld device across a patient body comprising:
a plurality of light sources (6)
the light sources (6) being disposed to be viewable by the user for visual reference of the speed at which the light appears to progress along the row
**characterized in that**
the plurality of light sources (6) are spaced apart in a row;
a light source driver for sequentially illuminating the light sources (6);
wherein the row of light sources are disposed on a flexible pad (5) for placing on the patient body adjacent to an area of the patient's body intended to be scanned or treated by the handheld device.

2. The apparatus of claim 1 further comprising a user controlled input apparatus for varying the speed at which the light appears to progress along the row.

3. The apparatus of claim 1 wherein the handheld device is a handheld external ultrasound treatment transducer and wherein the light sources are disposed to be viewable by the user of the external ultrasound treatment transducer for visual reference of the speed at which the light appears to progress along the row.

4. The apparatus of claim 3 further comprising a user controlled input apparatus for varying the speed at which the light appears to progress along the row.

5. The apparatus of claim 3 wherein the hand held device is a handheld external ultrasound lipoplasty transducer

## Patentansprüche

1. Vorrichtung zum Lenken der Abtastgeschwindigkeit, mit der ein Anwender ein Handgerät entlang eines Patientenkörpers bewegt, umfassend:
eine Vielzahl an Lichtquellen (6),
wobei die Lichtquellen (6) so angeordnet sind, dass sie von dem Anwender für eine visuelle Referenz der Geschwindigkeit sichtbar sind, mit der das Licht entlang der Reihe fortzuschreiten scheint,
**gekennzeichnet dadurch, dass**
die Vielzahl an Lichtquellen (6), in einer Reihe beabstandet sind, mit einer Lichtquellenansteuerung zum sequentiellen Beleuchten der Lichtquellen (6);
wobei die Reihe der Lichtquellen auf einem flexiblen Pad (5) zum Anbringen an den Patientenkörper angeordnet ist, benachbart zu einer Fläche des Patientenkörpers, die zum Abtasten oder zur Behandlung durch das Handgerät vorgesehen ist.

2. Vorrichtung nach Anspruch 1, ferner umfassend eine benutzergesteuerte Eingabevorrichtung zum Verändern der Geschwindigkeit, mit der das Licht entlang der Reihe fortzuschreiten scheint.

3. Vorrichtung nach Anspruch 1, wobei das Handgerät ein externer Ultraschallbehandlungs-Handtransducer ist und wobei die Lichtquellen angeordnet sind, dass sie von dem Anwender des externen Ultraschallbehandlungs-Transducers für die visuelle Referenz der Geschwindigkeit sichtbar sind, mit der das Licht entlang der Reihe fortzuschreiten scheint.

4. Vorrichtung nach Anspruch 3, ferner umfassend eine benutzergesteuerte Eingabevorrichtung zum Verändern der Geschwindigkeit, mit der das Licht entlang der Reihe fortzuschreiten scheint.

5. Vorrichtung nach Anspruch 3, wobei das Handgerät ein externer Ultraschall-Lipoplastik-Handtransducer ist.

## Revendications

1. Appareil pour guider la vitesse de balayage d'un utilisateur déplaçant un dispositif à main d'un bout à l'autre du corps d'un patient comprenant :
une pluralité de sources de lumière (6)
les sources de lumière (6) étant disposées pour être visibles par l'utilisateur pour une référence visuelle de la vitesse à laquelle la lumière semble progresser le long de la rangée
**caractérisé en ce que**
les plusieurs sources de lumière (6) sont espacées dans une rangée ;
un circuit de pilotage de sources de lumière pour illuminer séquentiellement les sources de lumière (6) ;
dans lequel la rangée de sources de lumière est disposée sur un coussinet souple (5) à placer sur le corps d'un patient adjacent à une zone du corps du patient destinée à être balayée ou traitée par le dispositif à main.

2. Appareil selon la revendication 1, comprenant en outre un appareil d'entrée commandé par l'utilisateur pour faire varier la vitesse à laquelle la lumière semble progresser le long de la rangée.

3. Appareil selon la revendication 1, dans lequel le dispositif à main est un transducteur de traitement par ultrasons externe à main et dans lequel les sources de lumière sont disposées pour être visibles par l'utilisateur du transducteur de traitement par ultrasons externe pour une référence visuelle de la vitesse à laquelle la lumière semble progresser le long de la rangée.

4. Appareil selon la revendication 3, comprenant en outre un appareil d'entrée commandé par l'utilisateur pour faire varier la vitesse à laquelle la lumière semble progresser le long de la rangée.

5. Appareil selon la revendication 3, dans lequel le dispositif à main est un transducteur de lipoplastie par ultrasons externe à main.
